# EUROPEAN PATENT SPECIFICATION

(11) **EP 1 480 515 B1**
(45) Date of publication and mention of the grant of the patent: **19.10.2005**
(21) Application number: 03743372.9
(22) Date of filing: 04.03.2003
(51) Int. Cl.: A01K 67/027

(54) **INBRED EMBRYONIC STEM-CELL DERIVED MICE**
DURCH INZUCHT ERZEUGTE VON EMBRYONALEN STAMMZELLEN ABGELEITETE MÄUSE
SOURIS ISSUES DE CELLULES SOUCHES EMBRYONNAIRES (ES) PURES

(30) Priority: 05.03.2002 US 362163 P
(43) Date of publication of application: 01.12.2004
(73) Proprietor: ARTEMIS Pharmaceuticals GmbH, 51063 Köln (DE)
(72) Inventor: KÜHN, Ralf, 80805 München (DE); RODE, Anja, 51065 Köln (DE); JAENISCH, Rudolph, Cambridge, MA 02142 (US); ZEVNIK, Branco, 51375 Leverkusen (DE)
(74) Representative: von Kreisler Selting Werner
(86) International application number: PCT/EP2003/002180
(87) International publication number: WO 2003/073843

(56) References cited:
- WO-A-00/15764
- EGGAN KEVIN ET AL: "Hybrid vigor, fetal overgrowth, and viability of mice derived by nuclear cloning and tetraploid embryo complementation" PROCEEDINGS OF THE NATIONAL ACADEMY OF SCIENCES OF USA, NATIONAL ACADEMY OF SCIENCE. WASHINGTON, US, vol. 98, no. 11, 22 May 2001 (2001-05-22), pages 6209-6214, XP002206043 ISSN: 0027-8424
- NAGY A ET AL: "DERIVATION OF COMPLETELY CELL CULTURE-DERIVED MICE FROM EARLY-PASSAGE EMBRYONIC STEM CELLS" PROCEEDINGS OF THE NATIONAL ACADEMY OF SCIENCES OF USA, NATIONAL ACADEMY OF SCIENCE. WASHINGTON, US, vol. 90, September 1993 (1993-09), pages 8424-8428, XP002938900 ISSN: 0027-8424
- BURDON T ET AL: "SUPPRESSION OF SHP-2 AND ERK SIGNALLING PROMOTES SELF-RENEWAL OF MOUSE EMBRYONIC STEM CELLS" DEVELOPMENTAL BIOLOGY, ACADEMIC PRESS, NEW YORK, NY, US, vol. 210, 1 June 1999 (1999-06-01), pages 30-43, XP000901724 ISSN: 0012-1606

## Description

### REFERENCE TO RELATED APPLICATIONS

This application claims priority to US provisional application 60/362,163 filed March 5, 2002. The contents of the prior application is hereby incorporated in its entirety.

An improved and reproducible method for the generation of ES mice from inbred ES cells, without the need to generate a chimeric mouse intermediate is provided. The inbred ES cells may be recombinant or genetically modified, resulting in ES mice that are transgenic. The method may also be used for the fast production of ES mice strains homozygous for a defined genetic alteration.

### BACKGROUND OF THE INVENTION

Genetically altered mice harboring specific transgenes or genetically engineered mutations have proven to be extremely useful tools for the analysis of gene function, the study of disease processes, and the discovery and testing of new therapies. However, current methods for generating recombinant mice are cumbersome, time consuming and expensive. A commonly used method of introducing targeted mutations or transgenes into the germ line of mice involves the use of embryonic stem (ES) cells as recipients of an engineered gene. ES cells are pluripotent cells that can be derived directly from the inner cell mass of blastocysts (Evans et al., (1981) Nature 292:154-156; Martin (1981) Proc. Natl. Acad Sci. USA 78:7634-7638; Magnuson et al., (1982) J. Embryo. Exp. Morph. 81:211-217; Doetchman et al., (1988) Dev. Biol. 127:224-227), disaggregated blastocysts (Eistetter, (1989) Dev. Gro. Differ. 31:275-282), and from primordial germ cells (Matsui et al., (1992) Cell 70:841-847; Resnick et al., (1992) Nature 359:550-551). Recombinant genes can be introduced into ES cells using any method suitable for gene transfer into cells, e.g., by transfection, cell fusion, electroporation, microinjection, DNA viruses, and RNA viruses (Johnson et al., (1989) Fetal Ther. 4 (Suppl. 1):28-39). The advantages of using ES cells include their ability to form permanent cell lines in vitro, thus providing an unlimited source of genetic material. Additionally ES cells are the most pluripotent cultured animal cells known. For example, when ES cells are injected into an intact blastocyst cavity or under the zona pellucida, at the blastocyst stage embryo, ES cells are capable of contributing to all somatic tissues including the germ line in the resulting animals. Besides the mouse, as described further below, ES-like cells have been isolated from rat (Iannaccone PM et al (1994) Dev Biol 163:288-292), pig (Chen LR et al. (1999) Theriogenology 52:195-212), bovine (Talbot NC et al. (1995) Mol Reprod Dev 42:35-52), rabbit (Schoonjans L et al. (1996) Mol Reprod Dev 45:439-443), primates (Thomson JA et al. (1995) PNAS 92:7844-7848) and human (Thomson JA et al (1998) Science 282:1145-1147).

Conventional procedures for the production of genetically engineered mice involve the use of genetically engineered ES cells to create genetically altered chimeric mice by either aggregation with diploid embryos or injection of engineered ES cells into diploid blastocysts, and subsequent introduction of the resulting chimeric embryos into pseudo-pregnant female mice (Capecchi, Science 244:1288-1292 (1989); Capecchi, Trends in Genetics 5:70-76 (1989)). The resulting chimeric mice are then bred to obtain mice that are heterozygous or homozygous for the desired genetic alterations. Despite the high success rate of this approach for generating recombinant mice, this method has serious limitations, particularly for generating large numbers of different recombinant mice carrying alterations in different genes in a high throughput setting, or for combining alterations in multiple genes in the same mouse strain. For example, the creation of a mouse with a homozygous mutation by the above approach requires a step of *in vitro* ES cell manipulation to target the gene of interest, followed by the production of a chimeric mouse. The chimeric founder animal is then bred to generate heterozygous progeny that are subsequently interbred to create mice homozygous for the desired alteration. Thus, the process of obtaining a homozygous mutant mouse requires at least three mouse generations, or 9 months of breeding time, to generate the desired mouse strain. These manipulations are complicated further in terms of breeding requirements if other mutations or transgenes are incorporated into the desired mutant mouse strain. Due to the lengthy time of breeding and the costs and effort of maintaining animals, it has become highly desirable, particularly in commercial or high throughput settings, to develop alternate methods to routinely generate genetically altered mice that do not require the production of a chimeric mouse intermediate.

Use of tetraploid embryos instead of diploid embryos for injection or aggregation with genetically altered ES cells is one approach that is used to circumvent the generation of a chimeric mouse intermediate and lengthy breeding steps (Nagy A et al. (1990), Development. 110:815-21; Misra RP et al. (2001) BMC Biotechnology 1:12). This method, termed "tetraploid complementation", takes advantage of the property that blastomeres may be readily made tetraploid (4N) by electrofusion of a two-cell embryo, and the resulting tetraploid cells have the capacity to replicate and form trophoblast and endoderm of the placenta and extraembryonic membranes, but fail to form fetal structures. On the other hand, ES cells have the capacity form fetal structures, but cannot form trophoblast and extraembryonic endoderm. Consequently, chimeric embryos formed by the introduction, either by injection or aggregation, of ES cells into tetraploid embryos successfully form normal concepti due to the complementary contributions of ES and tetraploid cells (Nagy et al., 1990; Nagy et al., PNAS (1993) 90:8424-8428; and James et al., Dev Biol (1995) 167(1):213-26). Thus, the method of tetraploid complementation appears to hold promise as a facile way to create nonchimeric ES cell-derived mice ('ES mice') in one step, without any additional breeding steps. However, in numerous studies attempting to apply the method of tetraploid complementation to the generation of ES mice the results have been highly variable at best, and for the most part viable and fertile ES mice have been produced in disappointingly low yield (Nagy A et al, (1990), *supra;* Nagy A et al. (1993) *supra;* Ueda O et al. (1995) Exp Anim. 44:205-10; Wang ZQ et al., (1997) Mech Dev. 62:137-45; PCT publication WO98/06834). In particular, it has proven especially difficult to obtain viable and fertile ES mice via tetraploid complementation using ES cells derived from inbred mouse lines (Nagy et al., 1993, *supra,* Ueda et al., 1995, *supra).* Also, in vitro genetic manipulation of ES cells or continued propagation of ES cells for prolonged times appeared to further reduce the efficiency to generate ES mice (Nagy et al., *supra).* Unfortunately, then, these collective results indicate that it may be commercially impractical to use tetraploid complementation for the routine generation of genetically engineered ES mice from inbred genetic backgrounds, due to extremely low efficiencies. This is especially problematic since inbred lines are the preferred lines to use for comparative studies of biological function, disease processes, or therapeutic efficacy, due to the constancy of genetic background in inbred strains.

Recently, Eggan et al. (Eggan K et al. (2001) PNAS 98:6209-6214) described a carefully controlled set of experiments comparing the behavior of inbred ES cells with F1 hybrid ES cells for the capacity to generate viable ES mice through tetraploid complementation. The F1 hybrid ES cells in this study were derived from outbred F1 progeny of crosses between different inbred mouse lines, e.g. 129 x C57BL/6, BALB/c x C57BL6, CBA x 129, 129/SvJ x 129/SV-CP and others. Remarkably, this study showed a reproducible and large difference in the capacity of inbred ES cells versus F1 hybrid ES cells to generate viable adult ES mice: inbred ES cells yielded viable adult ES mice at a low frequency of only 0-1.4%, depending on the strain used, whereas F1 hybrid ES cells yielded adult ES mice at an average frequency of 15%. Thus, in this study, inbred ES cells again proved impractical for use in routine generation of ES mice, even though F1 hybrid cells did work at a useful frequency. Although this study did not solve the problem of how to efficiently and routinely generate ES mice from inbred ES cells, it did demonstrate that differences presumably introduced by outbreeding in F1 progeny could somehow increase the efficiency of generation of ES mice by as much as 50-fold. Nonetheless, the complexity and mechanistic basis of this difference caused by outbreeding the F1 hybrid lines remained undefined, and therefore these results did not directly suggest a method to modify the isolation or manipulation of pure inbred ES lines such that inbred ES lines would have increased efficiency for the generation of ES mice via tetraploid complementation.

Several independent studies have focused on developing methods for improving the isolation and maintenance of undifferentiated pluripotent ES cell lines from a variety of sources. Leukemia Inhibitory Factor (LIF) has long been used as a key component of culture medium for isolation and propagation of undifferentiated ES cell lines (Pease S, et al. (1990) Dev Biol. 141:344-52). LIF is a soluble secreted protein factor that acts through a complex on recipient cells containing two receptors, gp130 and the low affinity LIF receptor, LIF-R. Response of cells to LIF appears to be mediated intracellularly by components of the STAT and MAPK signally pathways (Smith A and Burdon T, WO0015764). For propagation of ES cells LIF is typically provided either by a feeder layer of cells in the culture that express LIF, by preconditioning the culture medium through exposure to cells expressing LIF, or by adding recombinant LIF to the culture medium (Pease et al., *supra*). In addition to LIF other soluble protein-like factors have been characterized partially which appear to improve the isolation and maintenance of undifferentiated ES cells; Dani et al. have described a factor termed ESRF (Dani et al. (1998) Dev Biol 203:149-162; PCT application WO 9730151), and Schoonjans and Moreadith have described a conditioned medium with improved properties derived from recombinant rabbit fibroblasts expressing rabbit LIF (PCT application WO 0200847). In addition, Burdon et al. have presented data that inhibition of the MAPK signaling pathway, through the addition of ERK inhibitor PD098059 to the culture medium, enhances the growth of undifferentiated ES cells (Burdon et al. (1999) Dev. Biol. 210:30-43; PCT application WO 0015764). However, it has not been shown that such factors, which promote isolation or maintenance of ES cells in culture, can solve the problem of promoting tetraploid complementation of inbred ES cells for the generation of ES mice. For example, LIF has been used to maintain both inbred and F1 hybrid ES cells; yet, in one study, there was a 50-fold greater efficiency in the generation of adult ES mice using the F1 hybrid ES cells compared with the inbred ES cells (Eggan et al., *supra).* Consequently, there is no apparent direct relationship between factors that improve the isolation and/or maintenance of ES cells in culture compared to factors that might specifically improve the developmental potential of ES cells in tetraploid aggregates.

Thus, there remains a need in the art for improved and reproducible methods for the generation of genetically altered ES mice from genetically engineered inbred ES cells.

All references cited herein are incorporated in their entireties.

### SUMMARY OF THE INVENTION

The invention provides an improved and reproducible method for the generation of ES non-human mammals, preferably ES mice from inbred ES cells, without the need to generate a chimeric mouse intermediate. The method comprises the steps of propagating an inbred embryonic stem (ES) cell in a culture medium containing a predetermined amount of a ras/MAPK kinase pathway inhibitor. In preferred embodiments, the ras/MAPK inhibitor inhibits MEK1 and/or MEK2. A preferred MEK inhibitor is PD098095. Preferred non-human animals are rodents such as mice, rats, etc., with mice being most preferred. The invention is hereinafter described with reference to mice which is, however, not to be construed as to limit the invention.

The ras/MAPK-inhibited ES cells that are generated are introduced into a tetraploid embryo by injection into a tetraploid blastocyst or aggregation with a tetraploid morula, to generate an ES cell-complemented tetraploid embryo. The ES cell-complemented tetraploid embryo is transplanted into a female mouse, and viable ES mouse progeny are generated. In a preferred embodiment, the inbred ES cells are recombinant or genetically modified, in that they harbor a defined genetic alteration in their genome, such as a mutation, defined gene knock-out or knock-in, gene replacement and/or conditional knockout, and thus, the resulting ES mice are transgenic.

The method can be used for the fast production of ES mice strains homozygous for a defined genetic alteration. In this aspect of the invention, male ES cells harboring a defined genetic alteration are propagated under ras/MAPK pathway-inhibiting conditions to produce male (XY) cells and female (XO) cells. Tetraploid embryo complementation is performed with the male (XY) cells to generate viable male (XY) ES mice, and with isolated female (XO) cells to produce viable female (XO) ES mice. The male (XY) ES mice and the female (XO) ES mice are crossed to produce F1 progeny mice that are homozygous for the genetic alteration.

### DETAILED DESCRIPTION OF THE INVENTION

We have discovered that viable and fertile embryonic stem (ES) cell derived non-human mammals (ES mammals) such as ES cell derived-mice (ES mice) can be generated at much higher frequency than the previously described methods of using inbred ES cells and tetraploid complementation, when the inbred ES cells used for the tetraploid complementation have been propagated in a culture medium that contains an inhibitor of the ras/MAPK pathway. Thus, the invention provides an improved and reproducible method for the generation of ES mice from inbred ES cells, without the need to generate a chimeric mouse intermediate.

### Propagation of inbred embryonic stem (ES) cells

In the methods of the invention, an inbred ES stem cell is obtained, for example using an available cell line, or by generating primary ES cells using standard methods (see Hogan et al., in Manipulating the Mouse Embryo, CSHL press 1994 pp253-289). For example, male and female mice of inbred strains are allowed to naturally mate, and the ES cells from the blastocysts of fertilized mice are obtained. Any inbred strain can be used; preferred strains include C57BL/6, Balb/c, C3H, CBA, SJL, and 129SvEv/TAC (available from Janvier Le Genest-St-Isle, France and Taconic M&B, Denmark). The ES cells are cultured in medium and under standard conditions suitable for propagation of ES cells (Torres and Kuehn, Laboratory Protocols For Conditional Gene Targeting. (1997) Oxford University Press; Hogan et al. (1994); and Manipulating the Mouse Embryo, 2.edition, Cold Spring Harbor Laboratory Press, NY). Additionally, the culture medium is supplemented with an exogenously added inhibitor of the Raf/MEK/ERK signaling pathway (also referred to herein as the "ras/MAPK pathway"). The ras/MAPK pathway controls the activation of many cellular functions as diverse and (sometimes seemingly contradictory) as cell proliferation, cell-cycle arrest, terminal differentiation and apoptosis (see Murakami MS, Morrison DK., Sci STKE (2001) 99:PE30; and Peyssonnaux C, Eychene A., Biol Cell 2001 Sep;93(1-2):53-62).

Inhibitors of the ras/MAPK pathway that can be used are known in the art. Examples of small molecule inhibitors include ZM 336372 (N- [5- (e-Dimethylaminobenzamido) -2-methylphenyl]-4-hydroxybenzamide), an inhibitor of c-raf; 5-Iodotubercidin (Cas No. 24386-93-4), an inhibitor of ERK2; PD-98059 (2'-amino-3'-methoxyflavone; Cas No. 167869-21-8), an inhibitor of MEK (Alessi, D.R. et al. (1995) J. Biol. Chem. 270: 27489-27494); and U 0126 (bis [amino [(2-aminophenyl)thio] methylene] butanedinitrile; Cas No. 109511-58-2), also an inhibitor of MEK (Dudley, D.T. et al. (1995) Proc. Natl. Acad. Sci. USA 92: 7686-7689) (all aforementioned ras/MAPK pathway inhibitors are available from BIOMOL Research Labs, Plymouth Meeting, PA). An example of a protein inhibitor of the ras/MAPK pathway is Anthrax lethal factor which inhibits MEK (Duesbery NS, Vande Woude GF, J Appl Microbiol (1999) 87(2):289-93; Duesbery NS et al (2001) Proc Natl Acad Sci U S A. 98:4089-94). Preferred inhibitors inhibit MEK1 and/or MEK2. A particularly preferred inhibitor is PD-98059.

Additional Ras/MAPK pathway inhibitors can be identified using available assays. For example, inhibition of MEK can be detected by performing *in vitro* phosphorylation of an ERK:GST-fusion protein in the presence of various concentrations of a putative MEK inhibitor. Presence of the activated ERK is detected using Western blot and antibodies specific for active ERK (Said *et al*., Promega Notes, Number 69, 1998, p.6). Typically, small molecule inhibitors are used within the range of 10 nM to 100 mM. Preferred concentrations for PD98059 are in the range of 10-50 µM. Preferred concentrations of U0126 are in the range of 100 nM-10 µM. The optimal concentration of a particular inhibitor can be determined using routine experimentation. The concentration of the ras/MAPK inhibitor may be reduced once cell lines are established.

The culture medium is supplemented with an "exogenously added" ras/MAPK pathway inhibitor, meaning that the medium is supplemented with the inhibitor in a controlled manner. Typically, this is achieved by adding a known amount of a purified inhibitor to the culture medium to achieve a desired final concentration in the culture medium. In the case of protein inhibitors however, the culture medium may be supplemented with an "exogenously added" ras/MAPK pathway inhibitor by cells that express a recombinant inhibitor (e.g. recombinant feeder cells) in a sufficient amount to achieve ras/MAPK pathway inhibition. Thus, it is apparent from the foregoing examples that the term "exogenously added" does not encompass the situation where feeder cells in a conditioned medium, or the ES cells themselves, secrete an endogenously produced ras/MAPK inhibitor. Inhibitors of the ras/MAPK pathway used in the methods of the invention are typically small molecule or protein inhibitors such as the ones described above, but can also include other inhibitory agents, such as nucleic acid inhibitors (e.g. antisense, RNAi (see PCT WO 01/75164) etc.). The ES cells are cultured in the medium under conditions that promote proliferation. The cells may be passaged multiple times until the desired number of cells is obtained; they may then be used in tetraploid complementation, or frozen and stored for later use. ES cells propagated in the presence of a ras/MAPK pathway inhibitor are referred to herein as "ras/MAPK inhibited ES cells."

### Tetraploid Complementation

The ras/MAPK inhibited ES cells may be used for tetraploid complementation without further modification to generate cloned ES mice, or they may first be genetically modified, and then used for the production of transgenic ES mice (discussed further below).

Tetraploid embryos are generated using known methods (Wang et al., *supra;* WO98/06834; Eggan et al., *supra).* As an example, female mice are superovulated and mated. Fertilized zygotes are collected and cultured to obtain two-cell embryos, which are then electrofused to produce one-cell tetraploid embryos. The tetraploid embryos are cultured *in vitro* to the blastocyst stage. Ras/MAPK inhibited inbred ES cells are introduced into a tetraploid embryo using known methods such as aggregation with a tetraploid morula (Nagy et al., (1990), *supra*) or injection into a tetraploid blastocyst (Eggan et al., *supra).* Typically about 10-15 ES cells are introduced into each embryo, resulting what is termed herein as an "ES cell-complemented tetraploid embryo." Approximately 5-15 ES cell-complemented tetraploid embryos are implanted into recipient female mice, and allowed to develop to a point where they can survive ex-utero. The term "viable ES mouse progeny," is used herein to refer to an ES mouse generated by the above-described method that can survive at least 2 days after removal from the uterus of its recipient female mouse.

### Transgenic ES mice

Prior to tetraploid complementation, the ras/MAPK inhibited ES cells may be genetically modified to produce recombinant ES cells (i.e. that harbor a defined genetic alteration in their genome, such as a mutation, defined gene knock-out or knock-in, gene replacement and/or conditional knockout), and thus, the resulting ES mice are transgenic, which are used to generate transgenic ES mice. Alternatively, the ES cells themselves are derived from a transgenic or recombinant mouse, and thus are already "genetically modified". As another alternative, primary ES cells or existing ES cell lines are genetically modified prior to ras/MAPK inhibition. Methods for production of recombinant ES cells are well known in the art (Gene Knockout Protocols. In: Methods in Molecular Biology, Vol. 158. (2001) Eds MJ Tymms and 1. Kola, Humana Press, Totowa, New Jersey). Recombinant ES cells harbor altered expression (increased or decreased expression, including lack of expression) of one or more genes. Altered expression of genes in ES cells can be accomplished by gene knock-out, gene knock-in, and targeted mutations.

In one embodiment, the recombinant ES cells and resulting transgenic animals harbor a gene "knock-out", having a heterozygous or homozygous alteration in the sequence of an endogenous gene that results in a decrease of gene function, preferably such that gene expression is undetectable or insignificant. Knock-out cells are typically generated by homologous recombination with a vector comprising a transgene having at least a portion of the gene to be knocked out. Typically a deletion, addition or substitution has been introduced into the transgene to functionally disrupt it. The transgene can be a human gene (e.g., from a human genomic clone) but more preferably is an ortholog of the human gene derived from the transgenic host species. For example, a mouse gene is used to construct a homologous recombination vector suitable for altering an endogenous gene in the mouse genome. Detailed methodologies for homologous recombination in mice are available (see Capecchi, Science (1989) 244:1288-1292; Joyner *et al*., Nature (1989) 338:153-156). Procedures for the production of non-rodent transgenic mammals and other animals are also available (Houdebine and Chourrout, *supra;* Pursel *et al*., Science (1989) 244:1281-1288; Simms *et al*., Bio/Technology (1988) 6:179-183).

In another embodiment, the recombinant ES cell and the resulting transgenic animals harbor a gene "knock-in", having an alteration in its genome that results in altered expression (e.g., increased (including ectopic) or decreased expression) of the gene, e.g., by introduction of additional copies of a gene, or by operatively inserting a regulatory sequence that provides for altered expression of an endogenous copy of the gene. Such regulatory sequences include inducible, tissue-specific, and constitutive promoters and enhancer elements. The knock-in can be homozygous or heterozygous.

ES cells may also be used to produce transgenic nonhuman animals that contain selected systems allowing for regulated expression of a transgene. One example of such a system that may be produced is the cre/loxP recombinase system of bacteriophage P1 (Lakso *et al*., PNAS (1992) 89:6232-6236; U.S. Pat. No. 4,959,317). If a cre/loxP recombinase system is used to regulate expression of the transgene, animals containing transgenes encoding both the Cre recombinase and a selected protein are required. Such animals can be provided through the construction of "double" transgenic animals, e.g., by mating two transgenic animals, one containing a transgene encoding a selected protein and the other containing a transgene encoding a recombinase. Another example of a recombinase system is the FLP recombinase system of Saccharomyces cerevisiae (O'Gorman et al. (1991) Science 251:1351-1355; U.S. Pat. No. 5,654,182). In a preferred embodiment, both Cre-LoxP and Flp-Frt are used in the same system to regulate expression of the transgene, and for sequential deletion of vector sequences in the same cell (Sun X et al., (2000) Nat Genet 25:83-6).

The targeting constructs used to produce recombinant ES cells may be produced using standard methods, and preferably comprise the nucleotide sequence to be incorporated into the wild - type (WT) genomic sequence, and one or more selectable markers (Sambrook et al., 1989, Molecular Cloning: A Laboratory Manual, Second edition, CSHL press Cold Spring Harbor, New York; E. N. Glover (eds.), 1985, DNA Cloning: A Practical Approach, Volumes I and II; F.M. Asubel et al, 1994, Current Protocols In Molecular Biology, John Wiley and Sons, Inc.). The targeting construct may be introduced into the host ES cell using any method known in the art, such as microinjection, electroporation, retroviral-mediated transfer, sperm-mediated gene transfer, transfection, and calcium phosphate/DNA coprecipitation, among others. In a preferred embodiment, the targeting construct is introduced into host ES cells by electroporation (Potter H et al. (1994) PNAS 81:7161-7165). The presence of the targeting construct in cells is then detected by identifying cells expressing the selectable marker gene. For example, cells that express an introduced neomycin resistance gene are resistant to the compound G418.

Transgenic female ES mice derived from male ES cells can be generated for the fast generation of genetically altered inbred mouse strains. Male ES cells harboring genetic alterations can be propagated to generate ras/MAPK inhibited cells as described above. In rare cases, cell divisions lead to non-disjunction, thereby producing ES cells with an XO genotype (ES cells containing an X, but not a Y chromosome) among the progeny of the parental ES cells. ES cells with XY and XO genotypes may be distinguished using Y-specific markers. XY and XO ES cells are then used to produce transgenic ES mice, as described above. In contrast to humans (Turner-Syndrome), XO female mice are fertile. Therefore, ES mice with XY and XO genotypes are then mated, resulting in 25% offspring homozygous for a genetic alteration introduced into the parental male ES cell clone.

The transgenic ES mice produced using the methods of the invention can be used in a variety of applications, such as in genetic studies to elucidate signaling pathways or to identify additional genes involved in the same pathway that are also involved in disease progression. For example, two different transgenic mice, each harboring an altered gene known to be involved in cancer, can be mated to produce a double transgenic animal. The double transgenic animal is then used to determine the frequency and rate of cancer development. The identification of genes which accelerate malignant progression in a specific tissue, or which induce tumors in other tissues, provides further targets for therapeutic treatment.

Transgenic animals are also used as animal models of disease and disorders implicating defective gene function. They can also be used in drug development for *in vivo* testing of candidate therapeutic agents to evaluate compound efficacy and toxicity. The candidate therapeutic agents are administered to a transgenic animal having altered gene function and phenotypic changes are compared with appropriate control animals such as genetically modified animals that receive placebo treatment, and/or animals with unaltered gene expression that receive candidate therapeutic agent. Assays generally require systemic delivery of the candidate modulators, such as by oral administration, injection, etc. Following initial screening, a candidate therapeutic agent that appears promising is further evaluated by administering various concentrations of the compound to the transgenic animals in order to determine an approximate therapeutic dosing range.

### EXAMPLES

The following experimental section and examples are offered by way of illustration and not by way of limitation.

### 1. Derivation of wildtype and mutant C57BL/6 or 129SvEv/Tac inbred ES cell lines

ES cell derivation was essentially performed as described by Hogan et al. (Manipulating the mouse embryo, CSHL Press 1994, pp253-89), except that cell culture was performed at 39° C instead of 37° C. C57BL/6 (Janvier, France), 129SvEv/Tac (Taconic M&B, Denmark) or C57BL/6-APCMin (Jackson Laboratories, USA) male and female mice were mated to obtain blastocysts from fertilized females. The C57BL/6-APCMin mouse strain harbours a spontaneous mutation in the APC tumor suppressor gene (Su LK et al (1992) Science 256:668-670) and provides a genetic model for human hereditary colon cancer. Plug positive females were set aside, and 3 days later blastocysts were isolated by flushing their uteri. The blastocysts were further cultured overnight in CZB medium (Chatot et al. (1990) Biol. Reprod. 42:432-440) and then transferred into 12-well tissue culture plates (1 blastocyst per well), precoated with a monolayer of Mitomycin-C inactivated primary mouse embryonic fibroblasts, in standard ES cell culture medium ("standard conditions") (Torres and Kuehn, Laboratory protocols for conditional gene targeting, Oxford University Press 1997), or in standard medium supplemented with the MEK inhibitor PD 98059 (NEB Biolabs) (50 micromolar concentration, diluted from a 50 millimolar stock in DMSO stored at -20°C) or with the MEK inhibitor UO126 (10 micromolar concentration; NEB Biolabs). These cultures were incubated in a tissue culture incubator (Heraeus) for 6 days at 39°C in a 10% CO₂ atmosphere. The outgrown inner cell mass of each blastocyst was isolated with a pipette tip under low power magnification, transferred into a 50 microliter drop of Trypsin solution and incubated at 39°C for 5-10 minutes. This cell clump was further dissociated by pipetting, the cells were transferred into tissue culture dishes and further propagated at 39°C using standard ES cell culture conditions (Torres and Kuehn 1997, *supra*), with and without PD98059 (50 micromolar) or UO126 (10 micromolar). After the initial establishment of ES cell lines propagated in the presence of PD98059 (6 days after the first dissociation) the concentration of PD98059 was reduced to 25 micromolar, the concentration of UO 126 was reduced to 4 micromolar,and the cells were further propagated. The C57BL/6 line Bruce4 (Kontgen et al, (1993) Int Immunol. 5:957-964) was grown under standard conditions at 37°C.

The sex of the cell lines was determined through Southern blot hybridisation of genomic DNA using a detection probe (pY353) specific to a Y-chromosome specific repeat (Bishop CE and Hatat D. (1987) *Nucleic Acids Res* 15, 2959-2969).

For the karyotype analysis of ES cell clones, 4 x10⁶ cells were cultured for 1 hour in the presence of 0.2 µg/ml Demicolchicin (Sigma), trypsinised, resuspended in a hypotonic (0.56%) KCI solution and incubated for 8 minutes at room temperature. The swollen cells were sedimented by centrifugation, resuspended in freshly prepared fixative (3:1 mixture of ethanol/glacial acetic acid) and subsequently washed 2 times in fixative. Finally, the cells were resuspended in 0.5 ml fixative and dropped on glass slides precleaned with ethanol/acetic acid (19/1). After air drying, the metaphase spreads were stained for 5 minutes in a 2% solution of Giemsa's stain (Merck), washed in water, and air-dried. The chromosome numbers of 20 suitable metaphase spreads were counted at 1000x magnification under oil immersion.

These experiments resulted in 4 ES cell lines (2 male, 2 female) from 31 C57BL/6 blastocysts (13% efficiency) and 6 ES lines from 27 129SvEv/Tac blastocysts (22% efficiency) for ES cells grown under standard conditions. For cultures grown in media with PD 98059, 11 ES lines resulted from 18 C57BL/6 blastocysts (61% efficiency) and 9 ES lines were obtained from 12 129SvEv/Tac blastocysts (75% efficiency). From cultures grown in media with UO 126, 32 ES lines were obtained from 40 C57BL/6 blastocysts (80% efficiency). Thus, ES cell lines could be derived at a 5-fold higher efficiency in the presence of the MEK inhibitors PD 98059 or UO 126 (Table 1). Furthermore, 12 ES cell lines were generated from blastocysts of the C57BL/6-APCMin mutant mouse strain (36% efficiency).

**Table 1:**

| Derivation of ES Cell Lines | | | | | |
|---|---|---|---|---|---|
| **Cell line** | **Number of Blastocysts** | **Number of established ES lines** | **Efficiency to derive ES lines** | **Male ES lines** | **Female ES lines** |
| *conventional* | | | | | |
| C57BL/6 | 31 | 4 | 13% | 2 | 2 |
| 129SvEv/Tac | 27 | 6 | 22% | 5 | 1 |

| *with PD98059* | | | | | |
|---|---|---|---|---|---|
| C57BL/6 | 18 | 11 | 61% | 8 | 2 |
| 129SvEv.tac | 12 | 9 | 75% | 5 | 4 |

| *with UO126* | | | | | |
|---|---|---|---|---|---|
| C57BL/6 | 40 | 32 | 80% | 11 | 5 |
| C57BL/6 ApcMin | 33 | 12 | 36% | 4 | 8 |

### II. Injection of inbred ES cells into tetraploid blastocysts/ generation of inbred ES mice

Production of mice by tetraploid embryo complementation has been previously described (Eggan et al. (2001) PNAS 98:6209-6214). Briefly, embryo culture was carried out in microdrops on standard bacterial petri dishes (Falcon) under mineral oil (Sigma). Modified CZB media (Chatot et al, *Supra*) was used for embryo culture unless otherwise noted. Hepes buffered CZB was used for room temperature operations. After administration of hormones, superovulated B6D2F1females were mated with B6D2F1 males. Fertilized zygotes were isolated from the oviduct and any remaining cumulus cells were removed with hyluronidase. After overnight culture, two-cell embryos were electrofused to produce one cell tetraploid embryos using a CF150-B cell fusion instrument from BLS (Budapest, Hungary) according to the manufacturers instructions. Embryos that had not undergone membrane fusion within 1 hour were discarded. Embryos were then cultured *in vitro* to the blastocyst stage. For microinjection, 5-6 blastocysts were placed in a drop of DMEM with 15% FCS under mineral oil. A flat tip, piezo actuated microinjection-pipette with an internal diameter of 12-15 µm was used to inject 15 ES cells into each blastocyst. After recovery, ten injected blastocysts were transferred to each uterine horn of 2.5 days post coitum, pseudopregnant NMRI females that had been mated with vasectomized males. For cesarian derivation, recipient mothers were sacrificed at E 19.5 and pups were quickly removed. New-borns that were alive and respirating were cross-fostered to lactating females. Three days later, the litters were controlled, and pups alive by that time were counted as surviving pups.

Results: Two C57BL/6 derived ES cell lines (Bruce4 (Koentgen et al., supra) and ESAR-B6-8) established and grown under standard conditions were, as published, very inefficient in producing ES mice (Table 2; 0.67% respirating pups, 0% surviving pups). In contrast, new C57BL/6 ES lines established and grown in the presence of PD98059 produced significantly more alive pups (Table 2; 2.6% respirating pups, 0.57% surviving pups). ES cell lines established from 129SvEv/Tac blastocysts and grown in the presence of PD98059 also produced significantly more surviving pups (4.48%) upon tetraploid complementation as compared to three lines grown under standard conditions (0.85% surviving pups) (Table 2).

**Table 2:**

| EX Mouse Production From Inbred Cell Lines | | | | | | |
|---|---|---|---|---|---|---|
| **C57BL/6 ES Cell lines** | **Genetic background** | **Number of injected tetraploid blastocysts** | **Number of respirating pups** | | **Number of surviving pups** | |
| *w*/*o Substance* | | | | efficiency | | efficiency |
| Bruce4 | C57BL/6J | 220 | 1 | 0.45% | 0 | 0.00% |
| ESAR-B6-8 | C57BL/6J | 227 | 2 | 0.88% | 0 | 0.00% |
| Sum | | 447 | 3 | 0.67% | 0 | 0.00% |

| *with PD98059* | | | | | | |
|---|---|---|---|---|---|---|
| ESAR-B6-PD.13 | C57BL/6J | 180 | 4 | 2.22% | 0 | 0.00% |
| ESAR-B6-PD.3 | C57BL/6J | 372 | 8 | 2.15% | 1 | 0.27% |
| ESAR-B6-PD.4 | C57BL/6J | 329 | 11 | 3.34% | 4 | 1.22% |
| Sum | | 881 | 23 | 2.61% | 5 | 0.57% |
| | | | | | | |

| **129 ES Cell lines** | **Genetic background** | **Number of injected tetraploid blastocysts** | **Number of respirating pups** | | **Number of surviving pups** | |
|---|---|---|---|---|---|---|
| *w*/*o Substance* | | | | efficiency | | efficiency |
| 1295vEv/Tac | 129S6/SvE | 368 | 5 | 1.36% | 1 | 0.27% |
| ESAR-S6-15 | vTac | 136 | 2 | 1.47% | 1 | 0.74% |
| ESAR-S6-18 | 12956/SvE vTac | 321 | 10 | 3.12% | 5 | 1.56% |
| Sum | | 825 | 17 | 2.06% | 7 | 0.85% |

| *with PD98059* | | | | | | |
|---|---|---|---|---|---|---|
| ESAR-S6-PD.4 | 129S6/SvE vTac | 335 | 29 | 8.66% | 15 | 4.48% |

### III. Production of genetically engineered and mutant inbred ES mice

In order to generate inbred ES mice which harbour genetic alterations we pursued two different strategies. In the first protocol ES cell lines that were established and grown in media with UO 126 (see Example I) and which harbour the C57BL/6-APCMin mutation were injected into tetraploid blastocysts. These injection resulted in 12 respirating pups (1.9% efficiency) (Table 3). In the second protocol the C57BL/6 ES cell line ESAR-B6-PD.4 was genetically modified in a predetermined manner through homologous recombination using a gene targeting vector. This vector (Rosa(lacZ) knock-in) introduces a beta-galactosidase reporter gene in conjunction with a selectable hygromycin resistance gene into the endogenous Rosa26 locus of the ES cell genome (Seibler et al., Nucleic Acids Res. 31, e12, 2003). This gene targeting vector was electroporated into ESAR-B6-PD4 cells exactly as described (Seibler et al., Nucleic Acids Res. 31, e12, 2003) and hygromycin resistant ES cell colonies were selected, isolated and further expanded. The genomic DNA of resistant colonies was isolated and tested by Southern blot analysis for the occurrence of a homologous recombination event in one of the Rosa26 alleles. Cells from one of the recombined ES cell clones (ESAR-B6-PD.4-R9 A-F2) was injected into tetraploid blastocysts. These injections resulted in 4 respirating pups (2.47% efficiency) (Table 3).

**Table 3:**

| Production of Genetically Engineered and Mutant Inbred ES Mice | | | | |
|---|---|---|---|---|
| **C57BL/6 ES cell lines** | **Genetic background** | **Number of injected tetraploid blastocysts** | **Number of respirating pups** | |
| *with UO126* | | | | efficiency |
| ESAR-APC 3 UO | C57BL/6 J Apcmin | 405 | 11 | 2.72% |
| ESAR-APC 9 UO | C57BL/6 J Apcmin | 228 | 1 | 0.44% |
| Sum | | 633 | 12 | 1.90% |
| *with PD98059* | | | | |
| ESAR-B6-PD.4 R9 BG12 | C57BL/6 J | 162 | 4 | 2.47% |

### IV. Production of female transgenic ES mice from male ES cells for the accelerated generation of homozygous mouse mutants

Male ES cells harboring genetic alterations are propagated in culture medium with PD098059. In rare cases, cell divisions lead to non-disjunction, thereby producing ES cells with an XO genotype (ES cells containing an X, but not a Y chromosome) among the progeny of the parental ES cells. In a given ES cell culture the frequency of such 39XO cells is about 1-2%. These cells are isolated as pure clones by plating of the population at low density (1000 cells/culture dish) and further culture for 9 days until each single cell has formed a distinct colony of 1mm size (about 2000 cells). Using a pipette tip several hundred of these colonies are picked and distributed into wells of 96-well microtiter plates, prefilled with 50 microliter Trypsin solution. After a 10 minute incubation period the dissociated cells of each clone are further transferred into 96-well microtiter plates precoated with Mitomycin-C-inactivated embryonic feeder cells and cultured for several days in ES cell culture medium containing PD098059. Upon this initial culture phase the clones of each plate are split and distributed into two 96-well culture plates, one containing a layer of embryonic feeder cells while the other plate is precoated with a gelatine solution. Upon a 3 day expansion phase the culture medium in the plate containing the feeder layer is replaced by freezing medium and the plates are stored as frozen stock at -80°C. The second, gelatin coated plate is cultured for further two days until the ES cells reach confluency. Next, genomic DNA is isolated from these clones, digested with the restriction enzyme EcoRI, separated by agarose gel electrophoresis, and transferred onto nylon membranes by capillary transfer. These Southern blot membranes are hybridized with the Y-chromosome-specific 1.5 kb DNA probe from plasmid pY353 (Bishop, C.E. & Hatat, D. Molecular cloning and sequence analysis of a mouse Y chromosome RNA transcript expressed in the testis. *Nucleic Acids Res* 15, 2959-2969. (1987)). While the great majority of clones (98-99%) exhibit a strong Y chromosome specific signal, a minority of the clones (1-2%) shows no signal, presumably due to a spontaneous loss of the Y-chromosome which occurred in the progenitor of the cell which formed such a Y-negative colony. The latter clones are recovered from the frozen storage plate and further expanded in culture medium containing PD098059. To confirm that these clones underwent only a loss of the Y chromosome, their karyotype is characterized by chromosome counting of Giemsa stained metaphases as described in Example 1. Clones which exhibit a majority of metaphases with 39 chromosomes are defined as female cell lines with a 39,XO karyotype. These XO ES cells are then used to produce female transgenic ES mice through injection into tetraploid blastocysts, as further described below. Male ES mice with the same genotype as the XO ES females can be produced from the parental male ES cell population which was used to isolate the rare female cells. Male ES mice with a 40XY karyotype and female ES mice with a 39XO karyotype but the same genotype are then mated, resulting in 25% offspring homozygous for the genetic alteration which was introduced into the parental male ES cell line. This procedure to generate homozygous inbred mouse mutants involves only one breeding step and thus saves time as compared to the standard method via chimeric mice, which requires two breeding steps.

## Claims

1. A method of producing an embryonic stem cell derived-mouse (ES mouse) comprising:
a) obtaining ras/MAPK-inhibited ES cells derived from an inbred embryonic stem (ES) cell propagated in a culture medium containing a ras/MAPK kinase pathway inhibitor;
b) introducing ras/MAPK-inhibited ES cells obtained in (a) into a tetraploid embryo to generate an ES cell-complemented tetraploid embryo;
c) implanting the ES cell-complemented tetraploid embryo into a female mouse; and
d) generating a viable ES mouse progeny of the female mouse.

2. The method of claim 1 wherein the compound is an MEK inhibitor.

3. The method of claim 2 wherein the MEK inhibitor is PD098059.

4. The method of claim 1 wherein the ras/MAPK-inhibited cells are introduced into the tetraploid embryo by aggregation at the embryo's morula stage.

5. The method of claim 1 wherein the ras/MAPK-inhibited cells are introduced into the tetraploid embryo by injection at the embryo's blastocyst stage.

6. The method of claim 1 wherein the ras/MAPK-inhibited ES cells have a defined genetic alteration introduced by genetic modification.

7. The method of claim 6 wherein the ras/MAPK-inhibited ES cells are derived from a transgenic or recombinant mouse.

8. The method of claim 1 wherein the ES mouse harbors a genetic modification.

9. The method of claim 8 wherein the genetic modification is a mutation.

10. The method of claim 1 wherein:
in (a) the ras/MAPK-inhibited ES cells obtained are derived from a male (XY) ES cell that has been genetically modified *in vitro* to introduce a defined genetic alteration, and the ras/MAPK-inhibited ES cells comprise male (XY) cells and female (XO) cells;
prior to (b), the female XO cells are isolated from the male (XY) cells;
in (b), the ras/MAPK-inhibited male (XY) cells are introduced into a first tetraploid embryo to generate a male (XY) ES cell-complemented' tetraploid embryo, and additionally, the isolated ras/MAPK-inhibited female (XO) cells are introduced into a second tetraploid embryo to generate a female (XO) ES cell-complemented tetraploid embryo;
in steps (c) and (d), the male (XY) ES cell-complemented tetraploid embryo is implanted into a first female mouse to generate a viable male (XY) ES mouse progeny, and additionally, the female (XO) ES cell-complemented tetraploid embryo is implanted into a second female mouse to generate a viable female (XO) ES mouse progeny,
and wherein the method additionally comprises:
(e) crossing the male (XY) ES mouse progeny with the female (XO) ES mouse progeny to generate progeny inbred ES mice homozygous for the defined genetic alteration.

## Patentansprüche

1. Verfahren zur Herstellung einer von embryonischen Stammzellen abstammenden Maus (ES-Maus), umfassend:
a) Gewinnen von ras/MAPK-gehemmten ES-Zellen, die von einer ingezüchteten embryonischen Stammzelle (ES) stammen, die in einem Kulturmedium vermehrt wird, das einen Inhibitor des ras/MAPK-Kinase-Stoffwechselwegs enthält;
b) Einführen der in (a) erhaltenen ras/MAPK-gehemmten ES-Zellen in einen tetraploiden Embryo, so dass ein mit ES-Zellen komplementierter tetraploider Embryo entsteht;
c) Implantieren des mit ES-Zellen komplementierten tetraploiden Embryos in eine weibliche Maus; und
d) Erzeugen einer lebensfähigen ES-Maus-Nachkommenschaft der weiblichen Maus.

2. Verfahren gemäß Anspruch 1, wobei die Verbindung ein MEK-Inhibitor ist.

3. Verfahren gemäß Anspruch 2, wobei es sich bei dem MEK-Inhibitor um PD098059 handelt.

4. Verfahren gemäß Anspruch 1, wobei die ras/MAPK-gehemmten Zellen durch Aggregation im Morula-Stadium des Embryos in den tetraploiden Embryo eingeführt werden.

5. Verfahren gemäß Anspruch 1, wobei die ras/MAPK-gehemmten Zellen durch Injektion im Blastocysten-Stadium des Embryos in den tetraploiden Embryo eingeführt werden.

6. Verfahren gemäß Anspruch 1, wobei die ras/MAPK-gehemmten ES-Zellen eine durch genetische Modifikation eingeführte definierte genetische Veränderung aufweisen.

7. Verfahren gemäß Anspruch 6, wobei die ras/MAPK-gehemmten ES-Zellen von einer transgenen oder rekombinanten Maus stammen.

8. Verfahren gemäß Anspruch 1, wobei die ES-Maus eine genetische Modifikation beherbergt.

9. Verfahren gemäß Anspruch 8, wobei die genetische Modifikation eine Mutation ist.

10. Verfahren gemäß Anspruch 1, wobei:
in (a) die erhaltenen ras/MAPK-gehemmten ES-Zellen von einer männlichen (XY) ES-Zelle stammen, die in vitro genetisch so modifiziert wurde, dass eine definierte genetische Veränderung eingeführt wurde, und die ras/MAPK-gehemmten ES-Zellen männliche (XY) Zellen und weibliche (XO) Zellen umfassen;
vor (b) die weiblichen (X0) Zellen von den männlichen (XY) Zellen isoliert werden;
in (b) die ras/MAPK-gehemmten männlichen (XY) Zellen in einen ersten tetraploiden Embryo eingeführt werden, so dass ein männlicher (XY), mit ES-Zellen komplementierter tetraploider Embryo entsteht, und zusätzlich die isolierten ras/MAPK-gehemmten weiblichen (XO) Zellen in einen zweiten tetraploiden Embryo eingeführt werden, so dass ein weiblicher (XO), mit ES-Zellen komplementierter tetraploider Embryo entsteht;
in den Schritten (c) und (d) der männliche (XY), mit ES-Zellen komplementierte tetraploide Embryo in eine erste weibliche Maus implantiert wird, so dass eine lebensfähige männliche (XY) ES-Maus-Nachkommenschaft entsteht, und zusätzlich der weibliche (XO), mit ES-Zellen komplementierte tetraploide Embryo in eine zweite weibliche Maus implantiert wird, so dass eine lebensfähige weibliche (X0) ES-Maus-Nachkommenschaft entsteht;
wobei das Verfahren zusätzlich Folgendes umfasst:
(e) Kreuzen der männlichen (XY) ES-Maus-Nachkommenschaft mit der weiblichen (XO) ES-Maus-Nachkommenschaft, wobei ingezüchtete ES-Maus-Nachkommen entstehen, die für die definierte genetische Veränderung homozygot sind.

## Revendications

1. Une méthode de production d'une cellule souche embiyogénique dérivée d'une souris (souris ES) comprenant :
a) l'obtention de cellules ES au ras/MAPK inhibé dérivées d'une cellule souche (ES) consanguine propagée dans un milieu de culture contenant un inhibiteur de la voie de la kinase ras/MAPK ;
b) l'introduction des cellules ES au ras/MAPK inhibé obtenues à l'étape (a) dans un embryon tétraploïde pour générer un embryon tétraploïde complété par les cellules ES ;
c) l'implantation de l'embryon tétraploïde complété aux cellules ES dans une souris femelle ; et
d) la génération d'une progéniture viable de souris ES de la souris femelle.

2. La méthode de la revendication 1 dans laquelle le composé est un inhibiteur de MEK.

3. La méthode de la revendication 2 dans laquelle l'inhibiteur MEK est PD098059.

4. La méthode de la revendication 1 dans laquelle les cellules ES inhibées par ras/MAPK sont introduites dans l'embryon tétraploïde par l'agrégation à l'étape morula de l'embryon.

5. La méthode de la revendication 1 dans laquelle les cellules ES inhibées par ras/MAPK sont introduites dans l'embryon tétraploïde par injection à l'étape blastocyste de l'embryon.

6. La méthode de la revendication 1 dans laquelle les cellules ES inhibées par ras/MAPK ont une altération génétique introduite par modification génétique.

7. La méthode de la revendication 1 dans laquelle les cellules ES inhibées par ras/MAPK sont dérivées d'une souris transgénique ou recombinante.

8. La méthode de la revendication 1 dans laquelle la souris ES a une modification génétique.

9. La méthode de la revendication 8 dans laquelle la modification génétique est une mutation.

10. La méthode de la revendication 1 dans laquelle :
à l'étape (a) les cellules ES au ras/MAPK inhibé obtenues sont dérivées d'une cellule mâle (XY) ES qui a été génétiquement modifiée in vitro pour introduire une altération génétique définie et les cellules ES au ras/MAPK inhibé comprennent des cellules mâles (XY) et des cellules femelles (XO) ;
avant l'étape (b), les cellules femelles (XO) sont isolées des cellules mâles (XY) ;
à l'étape (b), les cellules mâles (XY) ES au ras/MAPK inhibé sont introduites dans une premier embryon tétraploïde pour générer un embryon tétraploïde complété aux cellules mâles (XY) ES et en plus les cellules isolées femelles (XO) au ras/MAPK inhibé sont introduites dans un deuxième embryon tétraploïde pour générer un embryon tétraploïde complété aux cellules femelle (XO) ES ;
aux étapes (c) et (d), l'embryon tétraploïde complété aux cellules ES mâles (XY) est implanté dans une première souris femelle pour générer une progéniture de souris ES viables mâles (XY) et en plus l'embryon tétraploïde complété aux cellules femelles (XO) est implanté dans une deuxième souris femelle pour générer une progéniture de souris ES femelles (XO)
et dans laquelle la méthode comprend en addition :
(e) croiser la progéniture de souris ES mâles (XY) avec la progéniture de souris ES femelles (XO) pour générer une progéniture de souris ES consanguines homozygotes pour l'altération génétique définie.
